# FASCICULE DE BREVET EUROPEEN

(11) **EP 1 125 941 B1**
(45) Date de publication et mention de la délivrance du brevet: **09.04.2003**
(21) Numéro de dépôt: 01400408.9
(22) Date de dépôt: 16.02.2001
(51) Int. Cl.: C07F 9/60, A61K 31/675, A61P 25/00

(54) **Dérivés d'acides 6-sulfamoyl-3-quinolyl phosphoniques, leur procédé de préparation et les compositions pharmaceutiques qui les contiennent**
6-Sulfamoyl-3-Quinolylphosphonsäurederivate, Verfahren zu ihrer Herstellung und sie enthaltende Arzneimittel
Derivatives of 6-sulfamoyl-3-quinolyl phosphonic acids, process for their preparation and pharmaceutical compositions containing them

(30) Priorité: 18.02.2000 FR 0002011
(43) Date de publication de la demande: 22.08.2001
(73) Titulaire: LES LABORATOIRES SERVIER, 92200 Neuilly sur Seine (FR)
(72) Inventeur: Cordi, Alex, 92150 Suresnes (FR); Desos, Patrice, 92400 Courbevoie (FR); Lestage, Pierre, 78170 La Celle Saint Cloud (FR)

(56) Documents cités:
- EP-A- 0 542 609
- EP-A- 0 640 612
- EP-A- 0 818 449
- US-A- 5 342 946

## Description

La présente invention concerne de nouveaux dérivés d'acides 6-sulfamoyl-3-quinolinyl phosphoniques, leur procédé de préparation et les compositions qui les contiennent.

La présente invention constitue une sélection du brevet EP0640612. Le brevet EP0640612 décrit des composés capables de s'opposer aux effets excitateurs et toxiques des aminoacides excitateurs (AAE) en bloquant l'activation initiale du récepteur à PAMPA (acide α-amino-3-hydroxy-5-méthyl-4-isoxazole-propionique) / kainate. Leur utilité est ainsi reconnue pour inhiber les phénomènes pathologiques, notamment neurotoxiques liés à l'hyperactivation des voies de neurotransmission aux aminoacides excitateurs. Cependant, la demanderesse a mis en évidence des problèmes graves de néphrotoxicité liée à l'utilisation de ces composés, comme par exemple pour l'acide (6,7-dichloro-2-oxo-1,2-dihydro-3-quinolinyl)phosphonique ainsi que cela a pu être montré par ailleurs pour d'autres antagonistes non-NMDA (N-méthyl-D-aspartate) de référence comme le 6-nitro-7-sulfamoyl-benzo[ƒ]quinoxaline-2,3-dione (NBQX) par exemple (Journal of Cerebral Blood Flow and Metabolism, 1994, 14, 251-261).

De façon surprenante, la demanderesse a découvert qu'un petit groupe de composés non décrits dans le brevet EP0640612 comporte non seulement des propriétés de puissants antagonistes non-NMDA mais qu'ils sont totalement dépourvus de néphrotoxicité associée. Ces composés sont donc nouveaux et sont de puissants agents thérapeutiques potentiels pour le traitement aigu mais également chronique des maladies neurologiques et psychiques impliquant ces aminoacides comme les maladies dégénératives telles que l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

Plus spécifiquement, la présente invention concerne les composés de formule (I) : 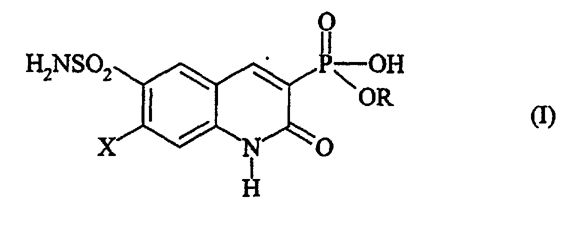 dans laquelle:
◆ X représente un atome de chlore ou de fluor ou un groupement trifluorométhyle,
◆ R représente un atome d'hydrogène ou un groupement 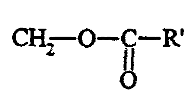
dans lequel
R' représente un atome d'hydrogène ou un groupement alkyle (C₁-C₆) linéaire ou ramifié ou phényle,
leurs isomères ainsi que leurs sels d'addition à une base pharmaceutiquement acceptable.

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels R représente un atome d'hydrogène.

Plus particulièrement, l'invention concerne les composés de formule (I) qui sont :
* l'acide (7-chloro-2-oxo-6-sulfamoyl-1,2-dihydro-quinolin-3-yl)-phosphorique,
* l'acide (7-trifluorométhyl-2-oxo-6-sulfamoyl-1,2-dihydro-quinolin-3-yl)-phosphonique,
* l'acide (7-fluoro-2-oxo-6-sulfamoyl-1,2-dihydro-quinolin-3-yl)-phosphonique.

Les isomères ainsi que les sels d'addition à une base pharmaceutiquement acceptable des composés préférés de l'invention font partie intégrante de l'invention.

L'invention s'étend également au procédé de préparation des composés de formule (I) caractérisé en ce que l'on utilise comme produit de départ un composé de formule (II) : 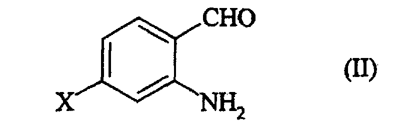 dans laquelle X est tel que défini dans la formule (I),
sur lequel on condense en présence d'une base comme la pyridine par exemple un composé de formule (III) : 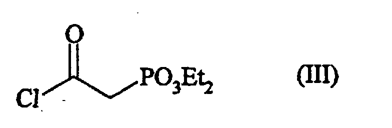 pour conduire au composé de formule (IV) : 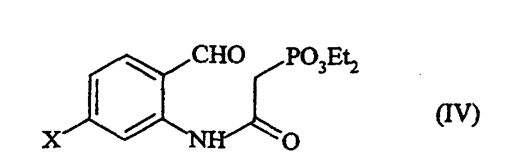 dans laquelle X est tel que défini précédemment,
que l'on cyclise en présence d'une quantité catalytique de pipéridine pour obtenir le composé de formule (V) : 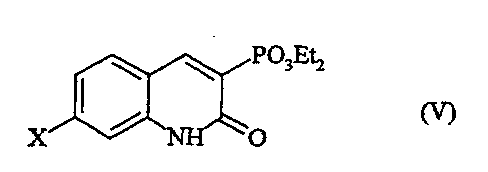 dans laquelle X est défini comme précédemment,
que l'on soumet à un mélange d'acide nitrique et d'acide sulfurique pour conduire au composé de formule (VI) : 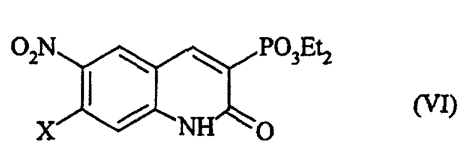 dans laquelle X est tel que défini précédemment,
qui est réduit en utilisant du charbon palladié en présence d'hydrogène ou du Fer en milieu hydroalcoolique pour obtenir le composé de formule (VII) : 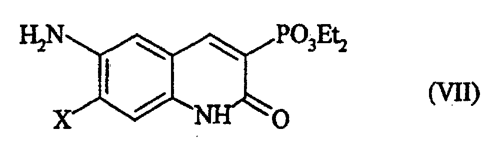 dans laquelle X est défini comme précédemment,
qui est soumis, après transformation en sel de diazonium correspondant, à l'action de dioxide de soufre en présence de CuCl₂ pour conduire au composé de formule (VIII) : 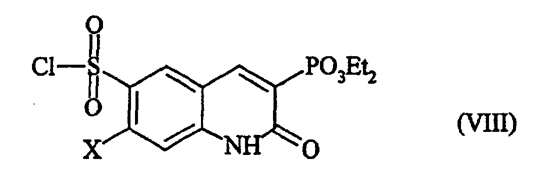 dans laquelle X est tel que défini précédemment,
qui est mis en présence d'une solution d'ammoniaque pour obtenir le composé de formule (IX) : 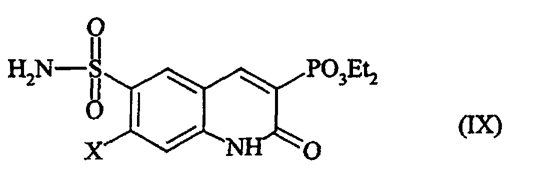 dans laquelle X est défini comme précédemment,
qui est déprotégé en présence de bromure de triméthylsilane en milieu acétonitrile pour conduire au composé de formule (I/a), cas particulier des composés de formule (I) : 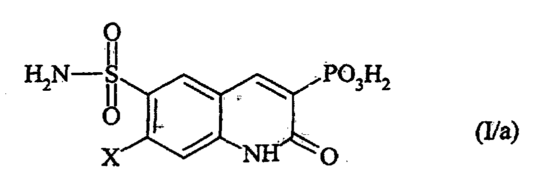 dans laquelle X est tel que défini précédemment,
que l'on peut mettre en réaction en milieu basique avec un composé de formule (X) : 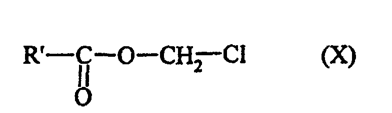 dans laquelle R' est tel que défini dans la formule (I),
pour conduire au composé de formule (I/b), cas particulier des composés de formule (I): 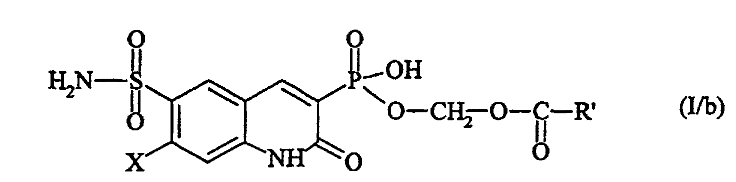 dans laquelle X et R' sont définis comme précédemment,
les composés de formule (I/a) et (I/b) formant l'ensemble des composés de formule (I), et pouvant être purifiée selon une technique classique de séparation, que l'on transforme, si on le souhaite en leurs sels d'addition à une base pharmaceutiquement acceptable et dont on sépare éventuellement les isomères selon une technique classique de séparation.

Les composés de l'invention présentent des propriétés pharmacologiques tout à fait intéressantes puisqu'ils sont de puissants inhibiteurs du récepteur AMPA, de surcroît sélectifs puisqu'ils ne touchent pas le récepteur NMDA et sont donc dépourvus de tous les effets secondaires décrits pour les antagonistes NMDA, et surtout dépourvus de la néphrotoxicité associée à nombre d'antagonistes AMPA/non-NMDA. L'utilisation de ces composés en tant qu'inhibiteurs des phénomènes pathologiques liés à l'hyperactivation des voies de neurotransmission aux aminoacides excitateurs sera ainsi particulièrement appréciée dans les traitements aigus et surtout chroniques des maladies neurologiques et psychiques impliquant ces aminoacides comme les maladies dégénératives telles que l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

La présente invention a également pour objet les compositions pharmaceutiques renfermant comme principe actif au moins un composé de formule (I) seul ou en combinaison avec un ou plusieurs excipients pharmaceutiquement acceptables.

Parmi les compositions pharmaceutiques selon l'invention, on pourra citer plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 50 mg et 1 g par 24 heures en 1 ou plusieurs prises.

Les exemples suivants illustrent l'invention et ne la limitent en aucune façon.

### EXEMPLE 1 : Acide (7-chloro-2-oxo-6-sulfamoyl-1,2-dihydro-3-quinolinyl) phosphonique

### Stade A : Acide [(5-chloro-2-formyl-phénylcarbamoyl)-méthyl]phosphonique diéthyl ester

A une solution de 2-amino-4-chloro-benzaldéhyde (6,18 g, 39,7 mmol) dans 170 ml de toluène anhydre on rajoute la pyridine (3,7 ml, 45,7 mmol) puis goutte à goutte une solution d'acide chlorocarbonylméthyl-phosphonique diéthyl ester (9,8 g, 45,7 mmol) dans 15 ml de toluène anhydre en maintenant la réaction à une température inférieure à 30°C. A la fin de l'addition, on agite 1 heure à température ambiante. La réaction est lavée plusieurs fois à l'eau puis avec une solution HCl 1N, puis de nouveau à l'eau. On termine par un lavage avec une solution aqueuse de NaCl saturée. La phase organique est séchée sur MgSO₄, on filtre, évapore et on obtient le produit brut attendu sous forme d'une huile orange. Le produit brut est engagé dans l'étape suivante.

### Stade B : Acide (7-chloro-2-oxo-1,2-dihydro-3-quinolinyl)phosphonique diéthyl ester

Dans un ballon surmonté d'un Dean-Stark on agite au reflux sous forte agitation pendant 4 heures la totalité du composé obtenu au stade A en solution dans 300 ml de toluène et 0,3 ml de pipéridine. On laisse cristalliser à température ambiante et on filtre le solide jaune pale obtenu.
*Point de fusion : 210-213°C*

| *Microanalyse élémentaire :* | | | | |
|---|---|---|---|---|
| | *C%* | *H%* | *N%* | *Cl%* |
| *calculé* | *49*,*46* | *4*,*79* | *4*,*44* | *11,23* |
| *trouvé* | *49*,*77* | *4*,*78* | *4*,*46* | *11*,*63* |

### Stade C : Acide (7-chloro-6-nitro-2-oxo-1,2-dihydro-3-quinolinyl)phosphonique diéthyl ester

A une solution de 55 ml d'acide sulfurique 96% refroidi dans un bain de glace on ajoute goutte à goutte 55 ml d'acide nitrique puis portion par portion l'acide (7-chloro-2-oxo-1,2-dihydro-quinolin-3-yl)-phosphonique diéthyl ester (14,7 g, 46,6 mmol) en maintenant la température inférieure ou égale à 5°C. A la fin de l'addition on poursuit l'agitation 15 minutes puis on retire le bain de glace et la réaction est amenée à température ambiante en 1 heure 30 environ. La solution est versée sur de la glace et le précipité est agité jusqu'à obtenir un solide filtrable. On filtre, lave à l'eau jusqu'à neutralité, sèche sous vide. Le solide est repris en suspension dans 210 ml d'éthanol au reflux, on laisse refroidir et on filtre pour obtenir après séchage le produit du titre.
*Point de fusion : 258-262°C*

| *Microanalyse élémentaire :* | | | | |
|---|---|---|---|---|
| | *C %* | *H %* | *N %* | *Cl %* |
| *calculé* | *43,29* | *3*,*91* | *7*,*77* | *9*,*83* |
| *trouvé* | *43*,*33* | *4*,*06* | *7*,*60* | *9*,*70* |

### Stade D : Acide (6-amino-7-chloro-2-oxo-1,2-dihydro-3-quinolinyl)phosphonique diéthyl ester

Une suspension du composé obtenu au stade C (7,0 g, 19,4 mmol), de Fer en poudre (10,8 g, 194 mmol), et de chlorure d'ammonium (10,4 g, 194 mmol) est agitée au reflux pendant 1 heure dans un mélange de 270 ml de méthanol et 90 ml d'eau. La suspension est filtrée à chaud sur célite et on rince plusieurs fois le solide avec du méthanol. Le filtrat est évaporé à sec et le résidu est repris en suspension dans de l'eau. On filtre le solide, on rince avec de l'eau et on sèche pour obtenir le produit du titre sous forme de cristaux oranges.
*Point de fusion : 255-260°C*

| *Microanalyse élémentaire :* | | | |
|---|---|---|---|
| | *C%* | *H%* | *N%* |
| *calculé* | *47*,*22* | *4*,*88* | *8*,*47* |
| *trouvé* | *47*,*06* | *4*,*99* | *8*,*08* |

### Stade E : Acide (7-chloro-6-chlorosulfonyl-2-oxo-1,2-dihydro-3-quinolinyl) phosphonique diéthyl ester

Une solution de 13,4 ml d'acide acétique et 2,25 ml d'eau est saturée en SO₂ par barbotage de SO₂ gaz pendant 15 minutes. Parallèlement on prépare à 5°C une solution du composé obtenu au stade D (3,34 g, 10,1 mmol) dans un mélange de 10 ml d'acide acétique glacial et 17 ml d'HCl concentré. A cette solution on ajoute goutte à goutte une solution de nitrite de sodium (767 mg, 11,11 mmol) préalablement dissout dans 5 ml d'eau et on agite la réaction 30 minutes à 5°C. A la solution saturée en SO₂ on rajoute CuCl₂.2H₂O (689 mg, 4,04 mmol) et la suspension obtenue est refroidie à 5°C. A cette dernière on rajoute goutte à goutte la solution du chlorure de diazonium préparée plus haut. Le mélange est agité 1 heure à 5°C puis 3 heures en laissant revenir à température ambiante. La réaction est versée sur de la glace et le précipité est filtré et rincé avec de l'eau. Après séchage on obtient le produit du titre sous forme de poudre jaune pale.
*Point de fusion : 190-200°C*

| *Microanalyse élémentaire :* | | | | | |
|---|---|---|---|---|---|
| | *C%* | *H%* | *N%* | *S%* | *Cl%* |
| *calculé* | *37,70* | *3*,*41* | *3*,*38* | *7*,*74* | *17,12* |
| *trouvé* | *38*,*04* | *3*,*47* | *3*,*40* | *7*,*74* | *17*,*16* |

### Stade F : Acide (7-chloro-2-oxo-6-sulfamoyl-1,2-dihydro-3-quinolinyl) phosphonique diéthyl ester

On agite une suspension du composé obtenu au stade E (1,26 g, 3,0 mmol) dans 18 ml d'ammoniaque 28 %. Après quelques minutes on observe un passage en solution. On poursuit l'agitation 30 minutes et on acidifie le milieu réactionnel avec HCl 4N. On ajoute sous agitation quelques ml d'acétate d'éthyle et il se produit une précipitation. Le précipité est filtré et séché sous vide pour conduire au produit du titre sous forme de poudre couleur crème.
*Point de fusion : 288-290°C*

### Stade G : Acide (7 -chloro -2 -oxo -6-sulfamoyl-1,2-dihydro-3-quinolinyl) phosphonique

A une suspension du composé obtenu au stade F (1,0 g, 2,53 mmol) dans 30 ml d'acétonitrile anhydre on ajoute 3,33 ml (25,3 mmol) de bromotriméthylsilane. On agite à reflux 1 heure et évapore à sec. Le résidu est séché sous vide et on le reprend dans du méthanol. On agite pendant 30 minutes la suspension qui devient de plus en plus épaisse. Après filtration du précipité blanc et rinçage avec un peu de méthanol puis d'éther on obtient le produit du titre.
*Point de fusion : > 300°C*

| *Microanalyse élémentaire :* | | | | |
|---|---|---|---|---|
| | *C%* | *H%* | *N%* | *S%* |
| *calculé* | *31*,*92* | *2*,*38* | *8,27* | *9*,*47* |
| *trouvé* | *31*,*76* | *2*,*52* | *7*,*92* | *9*,*18* |

### EXEMPLE 2 : Acide (7-trifluorométhyl-2-oxo-6-sulfamoyl-1,2-dihydro-3-quinolinyl)phosphonique

On procède comme dans l'Exemple 1 en remplaçant au stade A le 2-amino-4-chlorobenzaldéhyde par le 2-amino-4-trifluorométhyl-benzaldéhyde et en réalisant l'étape de réduction au stade D par le couple Pd-C/formiate d'ammonium au lieu du couple Fe/NH₄Cl en milieu hydroalcoolique.

### Stade A : Acide [(5-trifluorométhyl-2-formyl-phénylcarbamoyl)méthyl] phosphonique diéthyl ester

*Point de fusion : 62-64°C*

| *Microanalyse élémentaire :* | | | |
|---|---|---|---|
| | *C%* | *H%* | *N%* |
| *calculé* | *45,79* | *4,67* | *3,81* |
| *trouvé* | *45,89* | *4,66* | *3,76* |

### Stade B : Acide (7-trifluorométhyl-2-oxo-1,2-dihydro-3-quinolinyl)phosphonique diéthyl ester

*Point de fusion : 151°C*

| *Microanalyse élémentaire :* | | | |
|---|---|---|---|
| | *C%* | *H%* | *N%* |
| *calculé* | *48,15* | *4,33* | *4,01* |
| *trouvé* | *48,19* | *4,32* | *3,92* |

### Stade C : Acide (7-trifluorométhyl-6 -nitro -2-oxo-1,2-dihydro-3-quinolinyl) phosphonique diéthyl ester

*Point de fusion : 209-215°C*

| *Microanalyse élémentaire :* | | | |
|---|---|---|---|
| | *C%* | *H%* | *N%* |
| *calculé* | *42,65* | *3,58* | *7,11* |
| *trouvé* | *42,86* | *3,58* | *6,78* |

### Stade D : Acide (6-amino-7-trifluorométhyl-2 -oxo-1,2-dihydro-3-quinolinyl) phosphonique diéthyl ester

On agite à reflux pendant 1 heure un mélange de 490 mg (1,24 mmol) du composé obtenu au stade C, 650 mg (12,4 mmol) de formiate d'ammonium et 120 mg de Pd/C 10 % dans 50 ml d'éthanol. Le catalyseur est filtré sur membrane, le filtrat est évaporé à sec et le résidu est repris dans l'eau. On filtre la suspension, rince à l'eau, essore et sèche sous vide pour obtenir le produit du titre sous la forme d'un solide jaune.
*Point de fusion : 240-244°C*

| *Microanalyse élémentaire :* | | | |
|---|---|---|---|
| | *C%* | *H%* | *N%* |
| *calculé* | *46,16* | *4,43* | *7,69* |
| *trouvé* | *46,26* | *4,37* | *7,62* |

### Stade E : Acide (7-trifluorométhyl-6-chlorosulfonyl-2-oxo-1,2-dihydro-3-quinolinyl) phosphonique diéthyl ester

*Point de fusion : 165-171°C*

| *Microanalyse élémentaire :* | | | | | |
|---|---|---|---|---|---|
| | *C%* | *H%* | *N%* | *S%* | *Cl%* |
| *calculé* | *37,56* | *3,15* | *3,13* | *7,16* | *7,92* |
| *trouvé* | *37,54* | *3,20* | *3,18* | *7,05* | *7,97* |

### Stade F : Acide (7-trifluorométhyl-2-oxo-6-sulfamoyl-1,2-dihydro-3 -quinolinyl) phosphonique diéthyl ester

*Point de fusion : 258-259°C*

| *Microanalyse élémentaire :* | | | | |
|---|---|---|---|---|
| | *C%* | *H%* | *N%* | *S%* |
| *calculé* | *39,26* | *3,76* | *6,54* | *7,49* |
| *trouvé* | *39,54* | *3,65* | *6,46* | *7,35* |

### Stade G : Acide (7-trifluorométhyl-2-oxo-6-sulfamoyl-1,2-dihydro-3-quinolinyl) phosphorique

*Point de fusion : > 260°C*

| *Microanalyse élézmentaire :* | | | | |
|---|---|---|---|---|
| | *C%* | *H%* | *N%* | *S%* |
| *calculé* | *32,27* | *2,17* | *7,53* | *8,61* |
| *trouvé* | *32,45* | *2,01* | *7,64* | *8,96* |

### EXEMPLE 3 : Acide (7-fluoro-2-oxo-6-sulfamoyl-1,2-dihydro-3-quinolinyl) phosphonique

On procède comme dans l'Exemple 1 en remplaçant au stade A le 2-amino-4-chlorobenzaldéhyde par le 2-amino-4-fluoro-benzaldéhyde.

### Stade A : Acide [(5-fluoro-2-formyl-phénylcarbamoyl)méthyl]phosphonique diéthyl ester

Produit non isolé (huile) engagé tel quel dans le stade B.

### Stade B : Acide (7-fluoro-2-oxo-1,2-dihydro-3-quinolinyl)phosphonique diéthyl ester

*Point de fusion : 230-233°C*

### Stade C : Acide (7-fluoro-6-nitro-2-oxo-1,2-dihydro-3-quinolinyl)phosphonique diéthyl ester

*Point de fusion : 259-262°C*

| *Microanalyse élémentaire :* | | | |
|---|---|---|---|
| | *C%* | *H %* | *N%* |
| *calculé* | *45*,*36* | *4*,*10* | *8*,*14* |
| *trouvé* | *45*,*40* | *4*,*28* | *8*,*06* |

### Stade D : Acide (6-amino-7-fluoro-2-oxo-1,2-dihydro-3-quinolinyl)phosphonique diéthyl ester

*Point de fusion : 253-257°C*

| *Microanalyse élémentaire :* | | | |
|---|---|---|---|
| | *C%* | *H%* | *N%* |
| *calculé* | *49,69* | *5,13* | *8,91* |
| *trouvé* | *49,52* | *5,28* | *8,70* |

### Stade E : Acide (7-fluoro-6-chlorosulfonyl-2-oxo-1,2-dihydro-3-quinolinyl) phosphonique diéthyl ester

*Point de fusion : 159-160°C*

| *Microanalyse élémentaire :* | | | | | |
|---|---|---|---|---|---|
| | *C%* | *H%* | *N%* | *S%* | *Cl%* |
| *calculé* | *39,26* | *3,55* | *3,52* | *8,06* | *8,91* |
| *trouvé* | *39,74* | *3,73* | *3,74* | *7,85* | *8,59* |

### Stade F : Acide (7-fluoro-2 -oxo-6-sulfamoyl-1,2-dihydro-3-quinolinyl) phosphonique diéthyl ester

*Point de fusion : 266-268°C*

| *Microanalyse élémentaire :* | | | | |
|---|---|---|---|---|
| | *C%* | *H%* | *N%* | *S%* |
| *calculé* | *41,27* | *4,26* | *7,40* | *8,48* |
| *trouvé* | *41,17* | *4,48* | *7,17* | *8,34* |

### Stade G : Acide (7-fluoro-2-oxo-6-sulfamoyl-1,2-dihydro -3 -quinolinyl )phosphonique

*Point de fusion : >300°C*

| *Microanalyse élémentaire :* | | | | |
|---|---|---|---|---|
| | *C%* | *H%* | *N%* | *S%* |
| *calculé* | *33*,*55* | *2*,*50* | *8*,*69* | *9*,*95* |
| *trouvé* | *33*,*51* | *2*,*73* | *8*,*48* | *9*,*56* |

### ETUDE PHARMACOLOGIQUE

### EXEMPLEA : Inhibition du courant induit par une application de (R,S)-AMPA (10 µM) sur des ovocytes de Xenope injectés avec des ARNm de cortex de rat

Des ovocytes de Xenopes sont injectés avec 50 ng d'ARNm poly (A+) isolés de cortex cérébral de rat et incubés 2 à 3 jours à 18°C pour en permettre l'expression protéique. Les courants entrants induits par une application de (R,S)-AMPA (10 µM) sont mesurés dans un milieu de composition : NaCl (82,5 mM), KCl ( 2,5 mM), CaCl₂ (1 mM), MgCl₂ (1 mM), NaH₂PO₄ (1 mM), HEPES (5 mM), pH 7,4 par la méthode du voltage-clamp à 2 électrodes (potentiel = - 60 mV). Les produits de la présente invention sont appliqués de façon concentration dépendante 30 secondes avant et durant l'application de l'agoniste (R,S)-AMPA.

Leur capacité à inhiber le courant induit par le (R,S)-AMPA est déterminée par les valeurs d'IC50 (µM), représentant les concentrations inhibitant de 50 % le courant induit par une application de (R,S)-AMPA (10 µM).

Les composés de l'invention montrent d'excellentes propriétés inhibitrices avec des IC50 (µM) de l'ordre de 0,1.

### EXEMPLE B : Test des convulsions audiogènes chez la souris DBA/2

Chez la souris DBA/2 immature, des crises convulsives peuvent être déclenchées en soumettant cet animal à une stimulation sonore de haute intensité et de haute fréquence.

Les antagonistes des récepteurs au glutamate de type AMPA antagonisent ce type de convulsions de façon dose-dépendante (Chapman et al., Epilepsy Res., 1991, 9, 92-96). Ce test est utilisé pour étudier les effets anti-convulsivants des composés de la présente invention. Brièvement, des souris DBA/2 immatures (21-28 jours) sont exposées pendant 60 secondes à un bruit de 105 dB et de 18 kHz. Ceci entraîne l'apparition de convulsions cloniques. Les produits sous étude et le solvant sont administrés par voie i.p. 30 minutes avant le début du test sous un volume de 0,1 ml/10 g. Les ED50 (doses inhibant de 50 % l'incidence des convulsions) est déterminée pour chaque composé en utilisant la méthode de Litchfield et Wicoxon (J. Pharmacol. Exp. Ther., 1949, 96, 99-113).

Les composés de l'invention montrent une excellente capacité à inhiber les convulsions avec des ED50 de l'ordre de 3 mg/kg ip.

### EXEMPLE C : Test de néphrotoxicité chez le rat Fischer

L'évaluation de l'impact rénal des composés de la présente invention est réalisée chez le rat adulte male Fischer de 200-250 g. Des rats Fischer sont anesthésiés au pentobarbital (Nembutal®, 60 mg/kg i.p.). Quatre-vingt-dix minutes après l'induction de l'anesthésie, les composés sous étude sont administrés par voie intraveineuse aux doses de 3, 10 et 15 mg/kg. Vingt-quatre heures après administration, les animaux sont sacrifiés, le plasma est prélevé et la créatinémie et l'urémie sont mesurées. L'analyse statistique est réalisée à l'aide d'une analyse de variance à un facteur suivie d'un test de Newman-Keuls, en comparant les animaux traités et les animaux ayant uniquement reçu le véhicule.

Les composés de l'invention présentent une excellente tolérance rénale, aucun effet toxique n'étant obtenu pour des doses inférieures ou égales à 15 mg/kg iv.

### EXEMPLE D : Composition pharmaceutique

| | |
|---|---|
| 1000 comprimés dosés à 5 mg de l'acide (7-chloro-2-oxo-6-sulfamoyl-1,2-dihydro-3-quinolinyl)phosphonique (Exemple 1) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 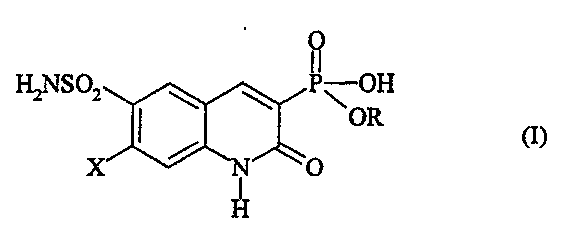 dans laquelle :
◆ X représente un atome de chlore ou de fluor ou un groupement trifluorométhyle,
◆ R représente un atome d'hydrogène ou un groupement 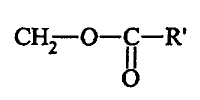
dans lequel
R' représente un atome d'hydrogène ou un groupement alkyle (C₁-C₆) linéaire ou ramifié ou phényle,
leurs isomères ainsi que leurs sels d'addition à une base pharmaceutiquement acceptable.

2. Composés de formule (I) selon la revendication 1 pour lesquels R représente un atome d'hydrogène, leurs isomères ainsi que leurs sels d'addition à une base pharmaceutiquement acceptable.

3. Composé de formule (I) selon la revendication 1 qui est l'acide (7-chloro-2-oxo-6-sulfamoyl-1,2-dihydro-3-quinolinyl)phosphonique, ses isomères ainsi que ses sels d'addition à une base pharmaceutiquement acceptable.

4. Composé de formule (I) selon la revendication 1 qui est l'acide (7-trifluorométhyl-2-oxo-6-sulfamoyl-1,2-dihydro-3-quinolinyl)phosphonique, ses isomères ainsi que ses sels d'addition à une base pharmaceutiquement acceptable.

5. Composé de formule (I) selon la revendication 1 qui est l'acide (7-fluoro-2-oxo-6-sulfamoyl-1,2-dihydro-3-quinolinyl)phosphonique, ses isomères ainsi que ses sels d'addition à une base pharmaceutiquement acceptable.

6. Procédé de préparation des composés de formule (I) selon la revendication 1 **caractérisé en ce que** l'on utilise comme produit de départ un composé de formule (II) : 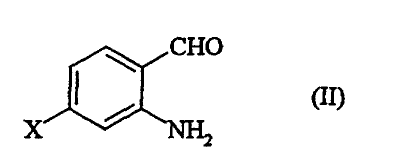 dans laquelle X est tel que défini dans la formule (I),
sur lequel on condense en présence d'une base comme la pyridine par exemple un composé de formule (III) : 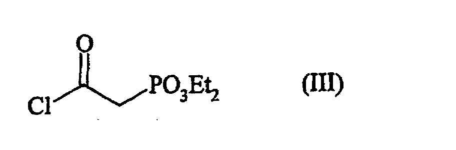 pour conduire au composé de formule (IV) : 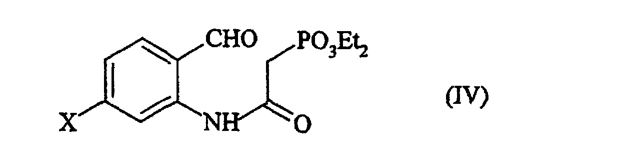 dans laquelle X est tel que défini précédemment,
que l'on cyclise en présence d'une quantité catalytique de pipéridine pour obtenir le composé de formule (V) : 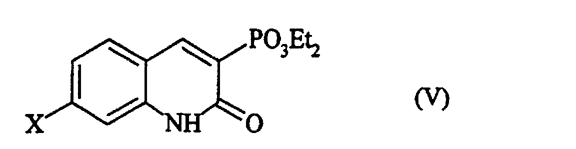 dans laquelle X est défini comme précédemment,
que l'on soumet à un mélange d'acide nitrique et d'acide sulfurique pour conduire au composé de formule (VI) : 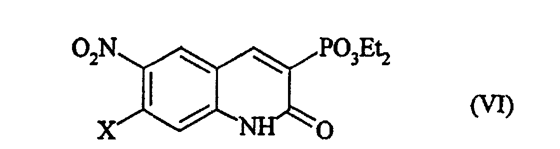 dans laquelle X est tel que défini précédemment,
qui est réduit en utilisant du charbon palladié en présence d'hydrogène ou du Fer en milieu hydroalcoolique pour obtenir le composé de formule (VII) : 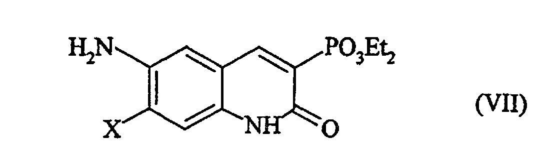 dans laquelle X est défini comme précédemment,
qui est soumis, après transformation en sel de diazonium correspondant, à l'action de dioxide de soufre en présence de CuCl₂ pour conduire au composé de formule (VIII) : 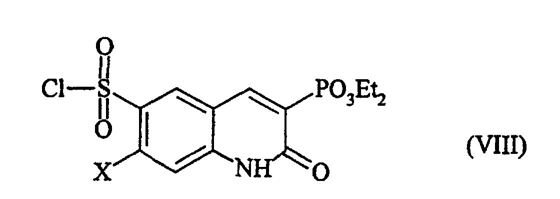 dans laquelle X est tel que défini précédemment,
qui est mis en présence d'une solution d'ammoniaque pour obtenir le composé de formule (IX) : 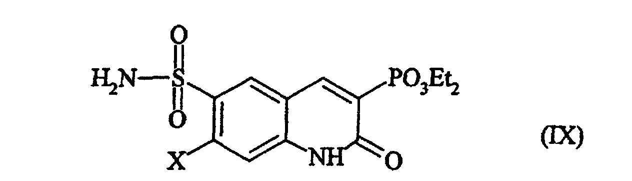 dans laquelle X est défini comme précédemment,
qui est déprotégé en présence de bromure de triméthylsilane en milieu acétonitrile pour conduire au composé de formule (I/a), cas particulier des composés de formule (I) : 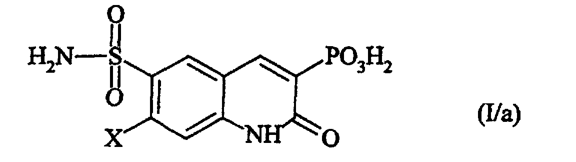 dans laquelle X est tel que défini précédemment,
que l'on peut mettre en réaction en milieu basique avec un composé de formule (X): 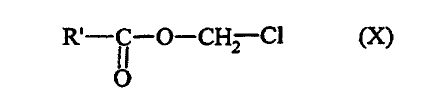 dans laquelle R' est tel que défini dans la formule (I),
pour conduire au composé de formule (I/b), cas particulier des composés de formule (I) : 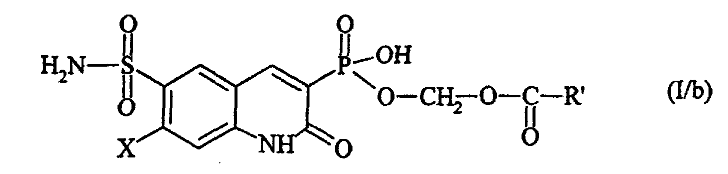 dans laquelle X et R' sont définis comme précédemment,
les composés de formule (I/a) et (I/b) formant l'ensemble des composés de formule (I), et pouvant être purifiés selon une technique classique de séparation, que l'on transforme, si on le souhaite en leurs sels d'addition à une base pharmaceutiquement acceptable et dont on sépare éventuellement les isomères selon une technique classique de séparation.

7. Compositions pharmaceutiques contenant comme principe actif au moins un composé de formule (I) selon l'une quelconque des revendications 1 à 5 ou un de ses sels d'addition à une base pharmaceutiquement acceptable, seul ou en combinaison avec un ou plusieurs excipients pharmaceutiquement acceptables.

8. Compositions pharmaceutiques selon la revendication 7 utiles pour la fabrication d'un médicament pour le traitement aigu ou chronique des phénomènes pathologiques liés à l'hyperactivation des voies de neurotransmission aux aminoacides excitateurs comme l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

## Claims

1. Compounds of formula (I) : 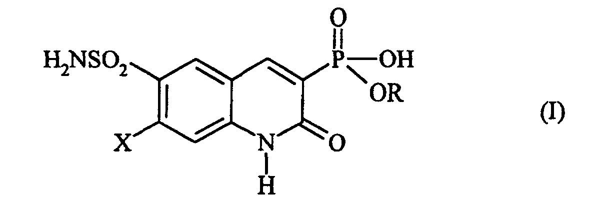 wherein:
◆ X represents a chlorine or fluorine atom or a trifluoromethyl group,
◆ R represents a hydrogen atom or a group 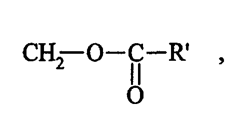
wherein R'
represents a hydrogen atom or a linear or branched (C₁-C₆)alkyl or phenyl group,
their isomers and addition salts thereof with a pharmaceutically acceptable base.

2. Compounds of formula (I) according to claim 1 wherein R represents a hydrogen atom, their isomers and addition salts thereof with a pharmaceutically acceptable base.

3. Compound of formula (I) according to claim 1 which is (7-chloro-2-oxo-6-sulphamoyl-1,2-dihydro-3-quinolyl)phosphonic acid, its isomers and addition salts thereof with a pharmaceutically acceptable base.

4. Compound of formula (I) according to claim 1 which is (7-trifluoromethyl-2-oxo-6-sulphamoyl-1,2-dihydro-3-quinolyl)phosphonic acid, its isomers and addition salts thereof with a pharmaceutically acceptable base.

5. Compound of formula (I) according to claim 1 which is (7-fluoro-2-oxo-6-sulphamoyl-1,2-dihydro-3-quinolyl)phosphonic acid, its isomers and addition salts thereof with a pharmaceutically acceptable base.

6. Process for the preparation of compounds of formula (I) according to claim 1, **characterised in that** there is used as starting material a compound of formula (II) : 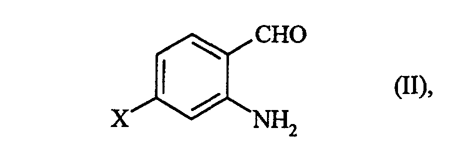 wherein X is as defined for formula (I),
which is condensed, in the presence of a base, such as, for example, pyridine, with a compound of formula (III) : 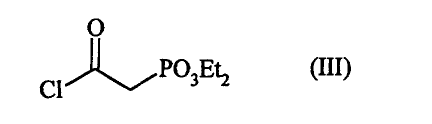 to yield a compound of formula (IV) : 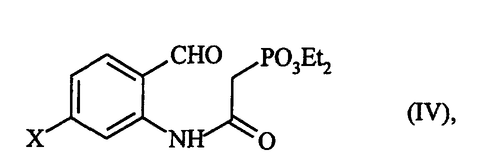 wherein X is as defined hereinbefore,
which is cyclised in the presence of a catalytic amount of piperidine to obtain a compound of formula (V) : 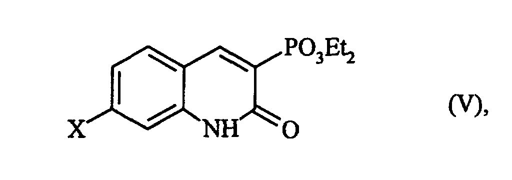 wherein X is as defined hereinbefore,
which is subjected to a mixture of nitric acid and sulphuric acid to yield a compound of formula (VI): 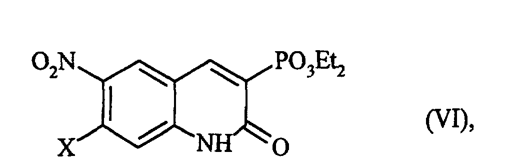 wherein X is as defined hereinbefore,
which is reduced using palladium-on-carbon in the presence of hydrogen or iron in an aqueous alcoholic medium to obtain a compound of formula (VII): 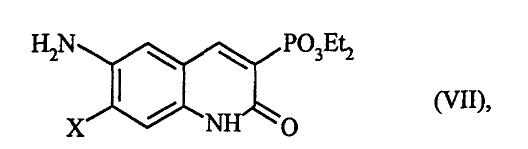 wherein X is as defined hereinbefore,
which is subjected, after conversion into the corresponding diazonium salt, to the action of sulphur dioxide in the presence of CuCl₂ to yield a compound of formula (VIII) : 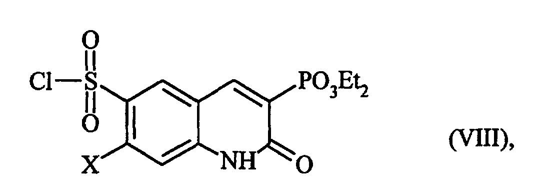 wherein X is as defined hereinbefore,
which is placed in the presence of ammonium hydroxide solution to obtain a compound of formula (IX) : 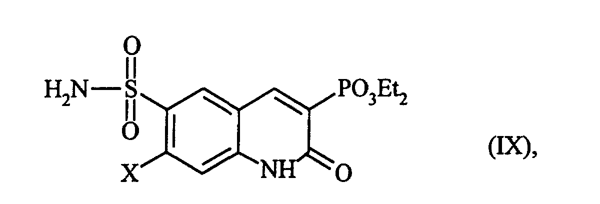 wherein X is as defined hereinbefore,
which is deprotected in the presence of trimethylsilane bromide in an acetonitrile medium to yield a compound of formula (I/a), a particular case of the compounds of formula (I) : 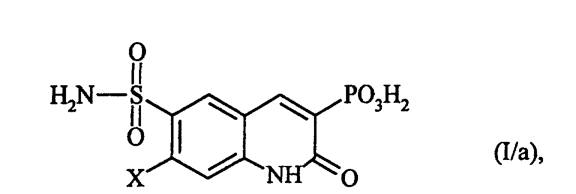 wherein X is as defined hereinbefore,
which may be reacted in a basic medium with a compound of formula (X) : 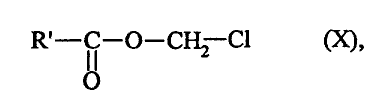 wherein R' is as defined for formula (I),
to yield a compound of formula (I/b), a particular case of the compounds of formula (I) : 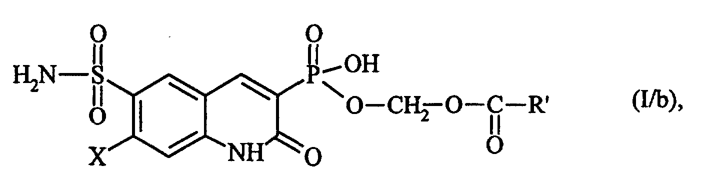 wherein X and R' are as defined hereinbefore,
which compounds of formulae (I/a) and (I/b) constitute the totality of the compounds of formula (I), and can be purified according to a conventional separation technique, are converted, if desired, into their addition salts with a pharmaceutically acceptable base, and separated, where appropriate, into their isomers according to a conventional separation technique.

7. Pharmaceutical compositions comprising as active ingredient at least one compound of formula (I) according to any one of claims 1 to 5 or an addition salt thereof with a pharmaceutically acceptable base, alone or in combination with one or more pharmaceutically acceptable excipients.

8. Pharmaceutical compositions according to claim 7 for use in the manufacture of a medicament for the acute or chronic treatment of pathological phenomena associated with hyperactivation of the neurotransmission paths to the excitatory amino acids, such as cerebrovascular accident, cerebral or spinal traumatism, epilepsy, chronic neurodegenerative diseases,such as Alzheimer's disease, schizophrenia, lateral amyotrophic sclerosis or Huntington's chorea.

## Patentansprüche

1. Verbindungen der Formel (I): 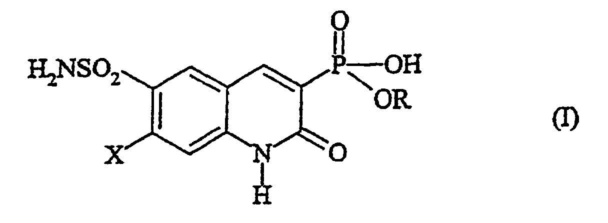 in der:
◆ X ein Chloratom, Fluoratom oder eine Trifluormethylgruppe,
◆ R ein Wasserstoffatom oder eine Gruppe 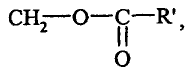 in der R' ein Wasserstoffatom oder eine geradkettige oder verzweigte (C₁-C₆)-Alkylgruppe oder Phenylgruppe darstellt, bedeuten,
deren Isomere sowie deren Additionssalze mit einer pharmazeutisch annehmbaren Base.

2. Verbindungen der Formel (I) nach Anspruch 1, worin R ein Wasserstoffatom darstellt, deren Isomere sowie deren Additionssalze mit einer pharmazeutisch annehmbaren Base.

3. Verbindung der Formel (I) nach Anspruch 1, nämlich (7-Chlor-2-oxo-6-sulfamoyl-1,2-dihydro-3-chinolinyl)-phosphonsäure, deren Isomere sowie deren Additionssalze mit einer pharmazeutisch annehmbaren Base.

4. Verbindung der Formel (I) nach Anspruch 1, nämlich (7-Trifluormethyl-2-oxo-6-sulfamoyl-1,2-dihydro-3-chinolinyl)-phosphonsäure, deren Isomere sowie deren Additionssalze mit einer pharmazeutisch annehmbaren Base.

5. Verbindung der Formel (I) nach Anspruch 1, nämlich (7-Fluor-2-oxo-6-sulfamoyl-1,2-dihydro-3-chinolinyl)-phosphonsäure, deren Isomere sowie deren Additionssalze mit einer pharmazeutisch annehmbaren Base.

6. Verfahren zur Herstellung der Verbindungen der Formel (I) nach Anspruch 1, **dadurch gekennzeichnet, daß** man als Ausgangsprodukt eine Verbindung der Formel (II) verwendet: 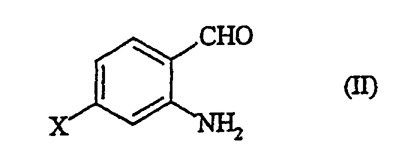 in der X die bezüglich der Formel (I) angegebenen Bedeutungen besitzt,
welche man in Gegenwart einer Base, wie beispielsweise Pyridin, mit einer Verbindung der Formel (III) kondensiert: 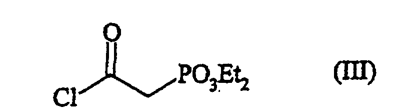 zur Bildung der Verbindung der Formel (IV): 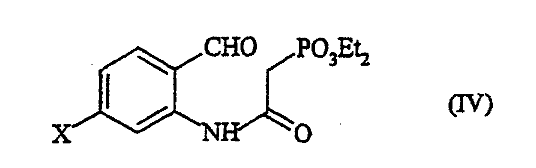 in der X die oben angegebenen Bedeutungen besitzt,
welche man in Gegenwart einer katalytischen Menge Piperidins cyclisiert zur Bildung der Verbindung der Formel (V): 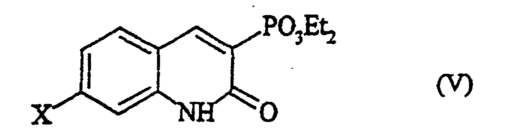 in der X die oben angegebenen Bedeutungen besitzt,
welche man mit einer Mischung aus Salpetersäure und Schwefelsäure behandelt zur Bildung der Verbindung der Formel (VI): 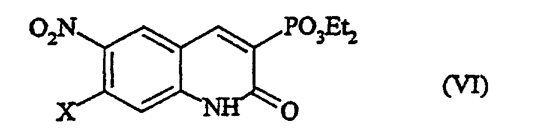 in der X die oben angegebenen Bedeutungen besitzt,
welche unter Verwendung von Palladium-auf-Kohlenstoff in Gegenwart von Wasserstoff oder Eisen in wäßrig-alkoholischem Medium reduziert wird zur Bildung der Verbindung der Formel (VII): 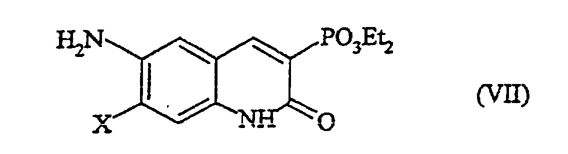 in der X die oben angegebenen Bededutungen besitzt,
welche nach der Umwandlung in das entsprechende Diazoniumsalz der Einwirkung von Schwefeldioxid in Gegenwart von CuCl₂ unterworfen wird zur Bildung der Verbindung der Formel (VIII): 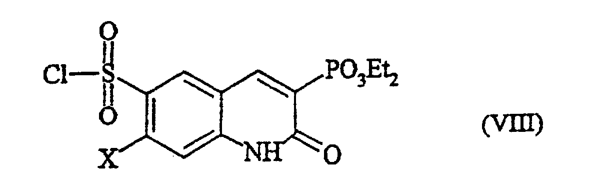 in der X die oben angegebenen Bedeutungen besitzt,
welche mit einer Ammoniaklösung behandelt wird zur Bildung der Verbindung der Formel (IX): 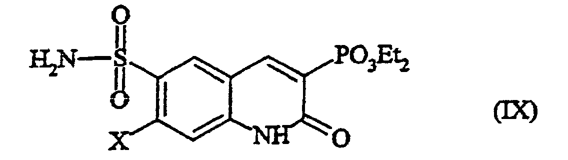 in der X die oben angegebenen Bedeutungen besitzt,
welche in Gegenwart von Trimethylsilan in Acetonitril als Medium von der Schutzgruppe befreit wird zur Bildung der Formel (I/a), einem Sonderfall der Verbindungen der Formel (I): 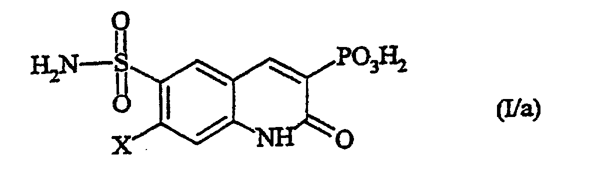 in der X die oben angegebenen Bedeutungen besitzt,
welche man in basischem Medium mit einer Verbindung der Formel (X) umsetzen kann: 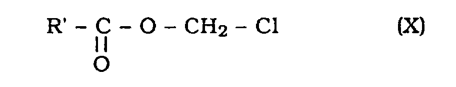 in der R' die bezüglich der Formel (I) angegebenen Bedeutungen besitzt,
zur Bildung der Verbindung der Formel (I/b), einem Sonderfall der Verbindungen der Formel (I): 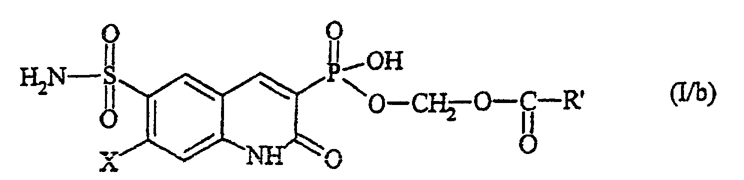 in der X und R' die oben angegebenen Bedeutungen besitzen,
wobei die Verbindungen der Formeln (I/a) und (I/b), die die Gesamtheit der Verbindungen der Formel (I) bilden, mit Hilfe einer klassischen Trennmethode gereinigt werden können, gewünschtenfalls in ihre Additionssalze mit einer pharmazeutisch annehmbaren Base umgewandelt werden können und die man gegebenenfalls mit Hilfe einer klassischen Trennmethode in ihre Isomeren trennen kann.

7. Pharmazeutische Zubereitungen enthaltend als Wirkstoff mindestens eine Verbindung der Formel (I) nach einem der Ansprüche 1 bis 5 oder eines ihrer Additionssalze mit einer pharmazeutisch annehmbaren Base allein oder in Kombination mit einem oder mehreren pharmazeutisch annehmbaren Trägermaterialien.

8. Pharmazeutische Zubereitungen nach Anspruch 7 nützlich für die Herstellung eines Arzneimittels zur Behandlung von akuten oder chronischen pathologischen Zuständen, die mit einer Hyperaktivierung der Neurotransmissionswege für anregende Aminosäuren verknüpft sind. wie Gehirnschlaganfällen, Gehirn- oder Rückenmarks-Traumata, der Epilepsie, chronischen neurodegenerativen Erkrankungen, wie der Alzheimerschen Krankheit, der Schizophrenie, der amyotrophischen Lateralsklerose oder der Huntington-Chorea.
